(19) **Europäisches Patentamt / European Patent Office / Office européen des brevets**

(11) **EP 2 881 456 A1**

(12) **EUROPEAN PATENT APPLICATION**

(43) Date of publication:
10.06.2015 Bulletin 2015/24

(51) Int Cl.:
*C11B 3/00* *(2006.01)*     *C12P 7/40* *(2006.01)*
*C12P 7/64* *(2006.01)*

(21) Application number: 13382494.6

(22) Date of filing: 04.12.2013

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **NATAC PHARMA, S.L.**
**28049 Madrid (ES)**

(72) Inventors:
• **Cela López, Jose Manuel**
  **28049 Madrid (ES)**
• **Quintela Fernández, Jose Carlos**
  **28049 Madrid (ES)**

(74) Representative: **Carlos Hernando, Borja**
**Garrigues IP, S.L.P.**
**Hermosilla, 3**
**28001 Madrid (ES)**

(54) **Enzymatic method for separating phytanic acid from fats or oils containing it and recovering unaltered products free of phytanic acid**

(57)     Enzymatic method for separating phytanic acid from fats or oils containing it and recovering unaltered products free of phytanic acid

The present invention relates to an enzymatic method that allows separating phytanic acid from any fat or oil containing it, as well as recovering fatty acids free of phytanic acid in an unaltered state on one hand, purified phytanic acid being obtained on the other hand with a purity by weight of at least 90%.

EP 2 881 456 A1

**Description**

Technical Field of the Invention

The present invention relates to an enzymatic method that allows separating phytanic acid from any fat or oil containing it, as well as recovering fatty acids free of phytanic acid in an unaltered state on one hand, purified phytanic acid being obtained on the other hand with a purity by weight of at least 90%.

The method of the present invention and the products obtained through same can therefore be applied in the food, cosmetics or pharmaceutical industries with regard to the use of fatty acids free of phytanic acid and of the fats or oils derived therefrom for human or animal consumption, as well as in the chemical industry with regard to the use of purified phytanic acid as an additive in processes for producing petroleum products, plant-derived fuels, cosmetics, pharmaceutical products, lubricants, insecticides, chemical markers and polymers.

Background of the Invention

It is well known in the state of the art that phytanic acid (3,7,11,15-tetramethylhexadecanoic acid or phytanic acid (PA), is present in high quantities in the diet in a number of products and fat or oil substances for human and animal consumption, such as fish and dairy products. Among others, marine-derived oils, which today are considered to be valuable products with a wide application in nutrition and food given their high long-chain omega-3 fatty acid content, can be mentioned. Other fats present in dairy products as well as animal fats from ruminants also have high phytanic acid contents from the microorganisms in the rumen.

Said foods make up a large part of the basic diet in humans and animals, in addition to being important components, in many cases, of food supplements, functional foods, etc.

However, phytanic acid unquestionably involves a high risk for human and animal health.

Although most foods contain less than 5 $\mu$g of phytanic acid/g, often those with the highest content generally exceed 1000 $\mu$g of phytanic acid/g, as can be seen in fish oil and the fat from dairy products (butter, cream, etc.). In addition to having the highest phytanic acid concentration, fish oils show higher toxicity because they interfere in breaking down (alpha oxidation) fatty acids, increasing phytanic acid levels (anti-thiamine effect) as a result. Diets with an identical phytanic acid content but of a different nature (fish diet and chlorophyll-rich diet) produce different phytanic acid contents in the organism consuming them, confirming that a fish diet gives rise to higher net phytanic acid levels (Hashimoto *et al.*, 2006).

Phytanic acid levels in commonly eaten foods are known from the state of the art: fish oils (1.0-4%), butter: (1.5-2.5%), cheese (0.7-1.5%), milk cream (0.5-1%), red meats (0.3%), lean fish (0.2%), fresh cheese (0.14%), ice-cream (0.1%), whole milk (0.1%), low-fat milk (0.05%).

However, taking into account that the diet in developed western countries is established at levels of 50-100 mg/day (Steinberg, 1983), it is calculated that dairy products are the first source of phytanic acid in such diet, exceeding 60% of the total that is consumed. In this sense, levels acquired through the following foods can be highlighted: cheese (20%), milk (20%) and butter (10%). Red meat represents 25-30% and fish represents only 3%. However, there are enormous differences (x6.7 fold) between vegetarian diets, Northern European and Scandinavian diets with respect to the Mediterranean countries, due to the difference in the consumption of foods such as red meats, butter, milk and fatty or blue fish. In these diets, the consumption of phytanic acid from dairy products is clearly higher. In all diets from developed western countries, dairy products (milk fat) represent the primary consumption of phytanic acid and are responsible for high phytanic acid plasma levels in humans, and accordingly, they are responsible for the increased risk of prostate cancer, breast cancer, colorectal cancer and non-Hodgkin lymphoma, *inter alia* (Ollberding *et al.*, 2013; Wright *et al.*, 2012; Allen *et al.*, 2008; Brown *et al.*, 1993).

Humans do not synthesize phytanic acid, but rather acquire it from the diet. In ruminants, the gut fermentation of plant materials releases phytol, a constituent of chlorophyll, which is then converted to PA, they do not accumulate significant amounts of phytanic acid as a result of consuming plant materials. However, they can obtain PA from ruminant fats, fish and dairy products.

On the other hand, humans with impaired phytanic acid catabolism can overaccumulate PA, which results in peripheral polyneuropathy, cerebellar ataxia, retinitis pigmentosa, anosmia and hearing loss. This can also result in cardiac arrhythmias, shortened metacarpals or metatarsals and ichthyosis (Watkins *et al.*, 2010).

For decades, epidemiological studies have suggested an association between fat, the risk of cancer and cardiovascular diseases. The most important works conducted in cancer tissue cultures and models with xenobiotics support the important role of several types of fat in modulation of the cancer phenotype. The elevated presence of phytanic acid in the primary food group targets in said epidemiological studies and their molecular markers have changed viewpoints on diet, fat and cancer, as well as neurodegenerative diseases (Alzheimer's disease), ophthalmological diseases, cardiovascular diseases, nephrological diseases, fertility diseases and immunological diseases, being attributed to phytanic acid an important role in the etiology, physiopathology and development of said diseases.

It seems that phytanic acid from the fat of fish and dairy products is fundamentally the cause of the risk of prostate cancer and of other types of cancer.

An epidemiological study conducted with 10,556 patients for 11 years (The Malmö Diet and Cancer Study, Wallström *et al.*, 2007) could demonstrate how consuming a diet rich in marine oils with omega-3 fatty acids could be the cause of a higher incidence in the risk of contacting cancer, although omega-3 fatty acids from fish (DHA and EPA) are also known to have qualities and mechanisms of protection against prostate cancer. Knowledge about phytanic acid has changed the mistaken viewpoint between the consumption of fish and prostate cancer, where all the studies shows a direct correlation between a high phytanic acid content and increase in the risk of prostate cancer, (Higashihara *et al.,* 2010; Wright *et al.*, 2012).

Over-consumption of red meat and dairy products, the major source of phytanic acid in human diet, has been demonstrated to be associated with prostate cancer, risk ratios of 1.3 or more having been observed (Xu *et al.*, 2005).

The James Buchanan Brady Urological Institute (Johns Hopkins Medicine) published in volume VI of 2003 the importance of a new marker, AMACAR ($\alpha$-methylacyl-CoA racemase) and other enzymes of the peroxisomal beta-oxidation pathway of phytanic acid and its high expression in prostate cancer and in the increased development of metastasis in said type of cancer as well as in other types, such as renal cell cancers, obesity and cancers related to insulin resistance (Wierzbicki-AS, 2007). There is evidence that the risk of prostate carcinoma increases due to the intake in the diet of foods rich in phytanic acid, and this risk additionally increases due to disturbances in AMACAR.

AMACAR is also strongly expressed in several other human carcinomas and their precursor lesions, implying a basic mechanistic role for this enzyme throughout the early stages of cancer formation (Mobley *et al.*, 2003).

The first phytanic acid catabolism step is mediated by $\alpha$-methylacyl-CoA racemase (AMACAR). AMACAR is an essential enzyme in two important metabolic pathways: elimination of branched-chain fatty acids (BCFAs) such as phytanic acid, and synthesis of bile acids and cholesterol. Patients with severe AMACAR deficiency suffer motor neuropathy and severe neonatal cholestasis with clotting disorders and poor fat absorption.

In another type of cases patients with AMACAR deficiencies showing an increase in phytanic acid levels, neuropathy and retinitis pigmentosa (Ferdinandusse *et al.*, 2000) have been described.

Total phytanic acid intake has been also associated with an increased risk of follicular lymphoma and small lymphocytic lymphoma/chronic lymphocytic leukemia. Studies in non-Hodgkin lymphoma provide support that total phytanic acid and phytanic acid-containing foods may increase the risk of this cancer (Olberding *et al.*, 2013).

Studies have demonstrated that high levels of saturated branched-chain fatty acids are deleterious to cells and animals, resulting in lipid accumulation and cytotoxicity, suggesting that the branched-chain saturated fatty acid (PA) is more cytotoxic than the straight-chain saturated fatty acid (palmitic acid) (Atshaves *et al.*, 2002).

Intracellular fatty acid-binding proteins (FABPs) reduce lipid accumulation induced by high levels of phytanic acid and other BCFAs but not saturated straight-chain fatty acids. These beneficial effects were displaced by means of the inhibition of the branched-chain fatty acid oxidation which was correlated with the enhanced toxicity of high branched-chain fatty acid levels. Phytanic acid oxidation was inhibited (74-100%), and the toxicity of phytanic acid was enhanced in the order L-FABP >> SCP-x > SCP-2 (Atshaves *et al.*, 2001). The importance of FABP expression for the progression of carcinomas (human breast cancer, colon cancer, renal cancer, skin cancer, etc.) is well-known. FABP suppression in cancer cells significantly inhibited proliferation and tumorigenicity, and FABPs are a candidate for a marker of tumor progression. Phytanic acid can be essential for the survival of several tumor cell lines.

In summary, phytanic acid is a potential public health risk factor because it can induce prostate cancer, breast cancer, colon cancer, lymphomas and other types of cancer, as well as neurological and visual disorders (Lloyd-MD *et al.*, 2008; Allen-NE *et al.*, 2008; Thornburg-G *et al.*, 2006; Xu-J *et al.*, 2005).

Alzheimer's disease, with the huge social and health impact on people in developed countries, has recently been reviewed and related with peroxisomal biogenesis defects (PBDs) (Lizard *et al.*, 2012), which are responsible, *inter alia*, for oxidation and for the increase in and accumulation of phytanic acid. Phytanic acid is an important marker of PBD and neurodegenerative diseases. Particularly, 2.5 fold increase in phytanic acid has been found in patients with Alzheimer's disease (Astartita *et al.*, 2010). Peroxisomes and mitochondria have an important effect on age-related diseases, neurodegenerative diseases, cardiovascular diseases and cancer.

Clinical observations have shown that phytanic acid in the diet is associated with a high toxicity level, despite the fact that the oxidative metabolism of phytanic acid in human beings is very fast (half the time of palmitic acid). At phytanic acid levels of about 5-10% of the total fatty acids in nerve tissue, phytanic acid provokes intense neuropathy and death. Even at much lower concentrations, phytanic acid is one of the molecules with the highest capacity to induce *in vivo* lipid oxidation.

Phytanic acid is directly toxic for the mitochondria and has strong atherogenic activity, rotenone-type activity, in the decoupling of complex I in oxidative phosphorylation in the inner mitochondrial membrane, resulting in the subsequent production of reactive oxygen species in the *in vivo* lipid peroxidation (Kahlert-S *et al.*, 2005) of polyunsaturated fatty acids in photoreceptors and neurons, increasing sensitivity to ischemia, to cardiovascular reperfusion injuries, low-density lipoprotein oxidation (LDLox), increasing macrophage inflammatory activity, reducing the energetic and metabolic activity (inhibiting oxidative phosphorylation) and inducing mitochondrial DNA mutation. This mitochondrial cytotoxic activity could explain why the photoreceptors, pigment epithelium, brain, neurosensory tissue, cerebellum and heart (Purkinje cells), bones, liver, ovaries, etc., tissues with high mitochondrial activity, are the first to be affected in patients with high phytanic acid concentrations.

Phytanic acid induces $Ca^{2+}$-mediated apoptosis in Purkinje cells (Powers-J.M *et al.* 1999) and sudden cardiac death in animal models. While omega-3 fatty acids reduce the risk of sudden death and the fatal risk of myocardial infarction, the phytanic acid contained in oils rich in omega-3 fatty acids increases the risk of sudden death (Mönning *et al.*, 2004). Phytanic acid causes ischemia, vascular smooth muscle apoptosis, is atherogenic and particularly cardiotoxic.

The mitochondrial activity is essential in fertilization and fetal development. Phytanic acid is found in sperm and in ova, being able to alter fertility and increase the miscarriage rate.

PA is involved in some human pathologies associated with retinitis pigmentosa, having discovered subsequently that it is connected with damage in the α-oxidation route and diseases with peroxisomal defects, characterized by an accumulation of PA in the blood and tissues.

Peroxisomal disorders are divided into three groups: A, B and C (Lazarow *et al.*, 1995; Wanders *et al.*, 1995). Group A or generalized are those in which there is a severe deficiency in peroxisomal biogenesis, such as such as cerebrohepatorenal syndrome of Zellweger and Infantile Refsum (phytanic acid storage) disease (IRD). The major phenotype of this category is one of dysmorphic facies, skeletal abnormalities, seizures, hypotonia and severe central nervous system lesions in the form of defective neuronal migration and abnormal central nervous system white matter. Group B, consisting primarily of rhizomelic chondrodysplasia punctata (RCDP) (Wierzbicki *et al.*, 2002; Wierzbicki, 2007), has demonstrable peroxisomes but displays multiple peroxisomal enzyme deficiencies. The major phenotype of RCDP consists of symmetrical shortening of the proximal limbs, cataracts and microcephaly. Group C are those disorders in which peroxisomes are morphologically intact and a single peroxisomal enzyme deficiency has been established, such as adrenoleukodystrophy, and its adult variant, adrenomyeloneuropathy.

These patients display severe central nervous system dysmyelinating/inflammatory demyelinating lesions and a non-inflammatory, dying back myelopathy, respectively. Unfortunately, group A and B patients typically die within a few months to years after birth, but there are also patients who survive longer (IRD and RCDP) (Powers *et al.*, 1999).

PA also affects some patients with RP, none associated with syndromes and other genetic diseases due to peroxisomal defects such as mitochondriopathies (Complex IV), COX deficiency, Leigh syndrome or Renal Fanconi syndrome (Fingerhut *et al.*, 1994; Wanders *et al.*, 2010).

PA elevations have been described in typical RP patients without syndromes. In normal controls, PA levels are almost undetectable (< 0.2 μg/ml; 0-33 μmol/l) whilst in RP patients with syndromes they are a hundred or a thousand times higher: 992-6400 μmol/l. High levels of PA are also found in non-systemic RP patients with anosmia and/or deafness-ataxia, although at significantly lower concentrations than in Refsum's syndrome: 38-192 μmol/l (Britton *et al.* 1989) and others syndromes with mitochondrial pathology and unusual RP (Pollitt *et al.* 1995). It is well known that PA significantly reduces the presence of plasmalogen levels, which plays a synergic pathogenic role in RP, resulting in irreversible damage of the mitochondrion function (Powers *et al.*, 1999).

Deficiencies in plasmalogens (30%) have also been found (dimethyl acetyl phosphatidylethanolamine) in RP for both simple and sporadic forms, recessive and dominant as well as in syndromes with peroxisomal biogenesis defects (PBDs) (Takemoto *et al.* 2003, Schaefer *et al.* 1995). Differences in concentrations of PA have been found associated with macular involvement. PA was found to be associated to a worse evolution, macular dystrophy and loss of visual acuity.

The exact mechanisms whereby phytanic acid is toxic for neurosensory and neural tissues, heart tissue, kidney tissue, liver tissue, intestinal tissue, smooth and striated muscle tissue, prostate tissue, breast tissue, sperm, lung tissue and the skeletal system are gradually becoming clearer. The best known mechanisms are associated with the decoupling of protonophoric action from the respiratory electron transport chain in the mitochondria and in cytoplasmic membranes (i.e., phototransduction in the retina). At a very low dose, phytanic acid is one of the molecules with the highest oxidative stress induction rates *in vivo.*

Phytanic acid (100 μM) drastically increases extracellular $Ca^{2+}$ influx and exerts strong depolarization of the mitochondria *in situ.* Furthermore, cell viability of cultured astrocytes is significantly reduced after 5 hours of exposure to phytanic acid. Isolated mitochondria become intensely de-energized by means of phytanic acid applied at low concentrations (5-20 μM). In energized mitochondria, de-energizing is primarily due to protonophoric activity of phytanic acid.

Furthermore, phytanic acid reduces phase 3 of respiration by means inhibiting the electron flow in the respiratory chain and inhibiting ADP/ATP exchange through the inner membrane. As an important functional consequence of these findings, mitochondria previously loaded with small amounts of $Ca^{2+}$ (100 nmol/mg of protein) are made highly sensitive to a rapid membrane permeability transition (MPT), even when only low phytanic acid concentrations (less than 5 μM) are applied.

Other findings indicate that phytanic acid-mediated $Ca^{2+}$ deregulation also involves GPR40 activation. Recent studies have elucidated that the toxic activity of PA and pristanic acid, branched-chain fatty acids associated with Refsum disease and other inherited peroxisomal disorders, is mediated by multiple mitochondrial dysfunctions, generation of reactive oxygen species and $Ca^{2+}$ deregulation via the InsP3-$Ca^{2+}$ signaling pathway. PA and pristanic acid-mediated intracellular $Ca^{2+}$ deregulation can involve the activation of free fatty acid receptor GPR40. It is therefore assumed that activation of GPR40 might be part of the signaling cascade of the toxicity of phytanic and pristanic acids (Kruska *et al.*, 2011) together with an increased ROS generation in neural tissues (Kahlert *et al.*, 2005).

Therefore there are many harmful effects on human health brought about by phytanic acid, which obviously translates into a need to provide mechanisms to separate the phytanic acid content from fats and oils containing it before being consumed by humans and animals, and/or before being used for other industrial purposes.

On the other hand, phytanic acid as such is an expensive and desired chemical that is very useful in industrial applications intended for obtaining, *inter alia,* petroleum products, plant-derived fuels, lubricants, insecticides, cosmetics and pharmaceutical products, as well as chemical markers and polymers.

A good example can be found in the production of biofuels. For ecotoxicity and commercial viability reasons, plant oil-derived fuel products, such as biofuels, have been developed. These biofuels, however, have a very high melting point and as a result must be mixed with gas oil and other petroleum and fossil fuel products at a very high percentage to enable being used as fuels in the industry and transport. Additives such as phytanic acid or the chemical derivatives thereof, such as phytane, have a remarkable value because they exhibit a very low melting point, significantly reducing the melting point of the plant-derived fuels, which raises the quality of plant-derived biofuels. Additionally, phytanic acid significantly reduces the cloud point and crystal formation in the biofuels that are produced as the temperature is reduced. Additionally, biodiesel tends to be converted into a gel at a temperature near 16°C. The addition of phytanic acid as an additive prevents the need to use mixtures with fossil fuels or to use it in smaller proportions. However, the high cost of phytanic acid limits its use as an additive in these applications in the chemical industry.

It would therefore be desirable to further have a method that allowed obtaining substantially pure phytanic acid in an efficient manner and with an improved yield for use in the chemical industry.

The state of the art discloses various methods for separating phytanic acid from fatty acids comprising it, *inter alia,* through transesterification reactions, fractionation with urea and supercritical fluid chromatography. Such methods are described in patent applications such as WO 01/10809, EP1157692, US2011033595, EP2464240 and WO2010118761.

Nevertheless, said methods require extreme chemical conditions, *inter alia*, isomerization and oxidation, in addition to high temperatures making the method as such more expensive and altering important physicochemical characteristics, nutritional composition, organoleptic properties and, in summary, the nature of fatty acids as well as the composition of isomers of the mixture in the raw material, fats and oils subjected to these methods. Additionally, said methods do not specifically separate phytanic acid from the products containing it, so the recovery and subsequent use thereof in the chemical industry is not viable. Finally, the methods of the state of the art are limited to being applied to specific raw materials.

The present invention provides a novel method for separating phytanic acid from any fat and/or oil containing it, for obtaining purified phytanic acid as well as fats and oils free of phytanic acid, said method being capable of overcoming the drawbacks associated with the methods known in the state of the art.

Brief Description of the Invention

For the purpose of the present invention, "pure or substantially pure phytanic acid" is understood as phytanic acid with a purity by weight of more than 90%, preferably a purity by weight between 95-99% with respect to the total weight of the sample. For the purpose of the present invention, the expression "fats and oils free of phytanic acid or substantially free of phytanic acid" is understood as those fats and oils the phytanic acid content of which is less than 50 $\mu$g/g, preferably less than 5 $\mu$g/g, with respect to the total weight of the sample.

For the purpose of the present invention, "unaltered" fats and oils are understood as those fats and oils the nutritional composition of which is identical to the nutritional composition of the sample before being subjected to the process of the invention with the exception of the phytanic acid content. For the purpose of the present invention, physicochemical characteristics of oils and fats are understood as those that allow defining fats and oils based on their physical properties, such as solubility, density, surface tension, and chemical properties, such as acidity, saponification value, peroxide value, fatty acid composition, etc. For the purpose of the present invention, organoleptic properties of oils and fats are understood as those that are detectable by the sense of taste and smell, i.e., the aroma and flavor of said fats and oils.

A first object of the present invention is therefore a method for separating phytanic acid from oils and fats containing phytanic acid, characterized by comprising a phytanic acid-specific enzymatic thioesterification step which results in obtaining a solid phase comprising phytanate-CoA and a liquid fraction comprising fatty acids free of phytanic acid.

Another object of the present invention is a method for separating phytanic acid from oils and fats containing phytanic acid, characterized by comprising a phytanic acid-specific enzymatic thioesterification step which results in obtaining a solid phase comprising phytanate-CoA and a liquid fraction comprising fatty acids free of phytanic acid. It further comprises a starting oil and fat hydrolysis step prior to the thioesterification step to obtain free fatty acids.

Another object of the present invention is a method which comprises, in addition to the steps described above, a separation step after the thioesterification step for separating the solid fraction containing phytanate-CoA and the liquid fraction containing fatty acids with a phytanic acid content less than 50 $\mu$g/g, obtained in the thioesterification step.

Another object of the present invention is a method for obtaining phytanic acid with a purity by weight of more than 90% which, in addition to comprising the aforementioned steps, is characterized in that the solid fraction of phytanate-CoA is subjected to non-enzymatic hydrolysis in alkaline medium for obtaining a liquid phase containing phytanic acid with a purity by weight of 90%.

Another additional object of the present invention is a method which comprises the steps described above for obtaining oils and fats with a phytanic acid content less than 50 $\mu$g/g, and which is characterized in that the liquid fraction is subjected to a re-esterification step for re-esterifying free fatty acids into mono-, di- and/or triglycerides.

Another additional object of the present invention is the use of fatty acids with a phytanic acid content less than 50 $\mu$g/g obtained through the method of the present invention for human and animal consumption in the food, pharmaceutical and cosmetics industries.

Another object of the present invention is the use of fish oils and phytanic acid contents of less than 50 $\mu$g/g obtained through the preceding method for human and animal consumption in the food, pharmaceutical and cosmetics industries.

Another object of the present invention is the use of milk and dairy products with a phytanic acid content less than 50 $\mu$g/g obtained through the method of the present invention for human and animal consumption.

Finally, another object of the present invention is the use of phytanic acid with a purity by weight of more than 90% obtained through the method of the present invention for use as an additive in the chemical, cosmetics and pharmaceutical industries.

Detailed Description of the Invention

The present invention has a dual objective: on one hand it seeks to obtain highly purified phytanic acid, and on the other it seeks to obtain fatty acids and/or fats and oils free of phytanic acid from fats and oils containing phytanic acid, in order to use said products in a number of both chemical-pharmaceutical applications with regard to phytanic acid, and in food, cosmetics and pharmaceutical applications with regard to fatty acids, as well as the fats and oils they incorporate or which are formed from said fatty acids free of phytanic acid.

The present invention thus provides an improved method for separating phytanic acid into oils and fats containing it, characterized by comprising a phytanic acid-specific enzymatic thioesterification step for the phytanic acid present in said samples.

Said method has the following advantages with respect to processes that are already known in the state of the art.

- Obtaining fatty acids substantially free of phytanic acid, i.e., fatty acids, such as DHA and/or EPA and/or mixtures thereof, with a phytanic acid content not considered harmful for human and animal health, i.e., with a phytanic acid content less than 50 $\mu$g/g, preferably less than 5 $\mu$g/g and which can be used as such or incorporated in derived products such as: new foods, dietetic foods, food supplements, cosmetics, etc.

- Obtaining fats and oils substantially free of phytanic acid for human and animal consumption, i.e., with a phytanic acid content not considered harmful for human and animal health, i.e., with a phytanic acid content less than 50 $\mu$g/g, preferably less than 5 $\mu$g/g, and which can be used as such or be incorporated in derived products such as: new foods, dietetic foods, food supplements, cosmetics, etc.

- Obtaining free fatty acids and/or fats and oils substantially free of phytanic acid for human and animal consumption which are, unaltered with respect to their nutritional composition, stereoisomeric configuration, physicochemical characteristics and organoleptic properties.

- Selectively separating phytanic acid from samples of oils and fats containing it and recovering purified phytanic acid with a purity by weight of more than 90%, preferably a purity by weight between 95-99%, for use in the chemical industry, food industry, pharmaceutical industry, cosmetics industry, petrochemical industry, etc.

One of the main features and advantages of the method object of the present invention is that it is intended for selectively separating phytanic acid from any type of fats and oils, i.e., it is a method that can be applied to any type of fat and/or oil containing phytanic acid.

Therefore there are innumerable fats and oils that can be subjected to the method of the present invention. Generally, the method would be applied to animal, marine, vegetable and microbial fats and/or oils, but the application thereof for separating phytanic acid in marine sediments, phytoplankton, dinoflagellates, diatoms, algae and microalgae, is also contemplated.

Said fats or oils with a phytanic acid content may or may not be intended for human and/or animal consumption, may or may not be found in a purified state, may or may not have been subjected to a refining process, and may be part of derived or processed food products.

Examples of animal fats and/or products derived therefrom are: milk cream, butter, fish oils, whale fat, semi-solid and solid fat obtained from tallow, lard, bacon fat, marine oils such as oil from krill, algae, ground phytoplankton, diatoms, dinoflagellates, fish oil (cod liver oil, herring fish oil, sardine fish oil, salmon fish oil, anchovy fish oil, tuna fish oil,...), whale fat, etc.

A preferred embodiment of the present invention contemplates applying the method of the present invention to fish oils with omega-3 fatty acid concentrations less than 65% by weight with respect to the total fatty acids present in the sample.

Examples of vegetable oils are: palm oil, rapeseed oil, mustard seed oil, peanut oil, coconut oil, cottonseed oil, palm kernel oil, corn oil, sunflower seed oil, olive oil, and seed oils, such as sesame seed oil.

Examples of microbial oils are: marine sediments and bacterial extracts of *Fibrobacter succinogenes (Bacteroides succinogenes), Treponema (Borrelia) sps., Selenomonas ruminantium*, *Halobacterium sp.*, *Acinetobacter sp.* strain PHY9, *Pseudomonas nautica* (IP85/617), *Marinobacter sp.* strain CAB (DSMZ 11874), *and Marinobacter hydrocarbonoclasticus* (ATCC 49840).

Examples of refined fats and oils are: purified fish (sardine, anchovy, tuna, herring, salmon, cod, etc.) oils rich in omega-3. Examples of fats and oils that are part of derived or processed products are: dairy products such as: butter, milk cream and cream, etc.

The fats or oils that can be used as a starting material in the method of the present invention contain phytanic acid primarily in the form of triglyceride, although it can also be presented in small proportions as free fatty acid, phospholipids, etc., the phytanic acid content being more than 10 $\mu$g/g, as is the case of skim milk, but there being no limitation as to a maximum phytanic acid content in the sample to be treated.

As a result of the method of the present invention, the following products/fractions are obtained:

- Free fatty acids with a phytanic acid content less than 50 $\mu$g/g, preferably less than 5 $\mu$g/g.
- Phytanic acid in the form of free fatty acid with a purity by weight of at least 90%, preferably a purity by weight between 95 and 99%, with respect to the total weight of the sample.
- Fats or oils with a phytanic acid content less than 50 $\mu$g/g, preferably less than 5 $\mu$g/g.
- Fish oils with a phytanic acid content less than 50 $\mu$g/g, and preferably less than 5 $\mu$g/g, furthermore having an omega-3 fatty acid concentration less than 65% by weight with respect to the total fatty acids present in the sample.
- Milk and dairy products with a phytanic acid content less than 50 $\mu$g/g, preferably less than 5 $\mu$g/g.

The method of the present invention thus comprises an essential phytanic acid-specific enzymatic thioesterification step for the phytanic acid present in the sample to be treated, subsequently obtaining a solid fraction comprising phytanate-CoA and a liquid fraction comprising fatty acids substantially free of phytanic acid, i.e., with a phytanic acid content less than 50 $\mu$g/g, preferably less than 5 $\mu$g/g.

The method of the present invention is also characterized by comprising, prior to the phytanic acid-specific enzymatic thioesterification step, a starting material hydrolysis step to obtain free fatty acids which allows efficiently performing the subsequent phytanic acid-specific thioesterification step.

Additionally, the method of the present invention comprises, after the specific enzymatic thioesterification step, a separation step for separating the solid and liquid phases obtained in the prior thioesterification step for the purpose of separating the phases containing phytanic acid and fatty acids free of phytanic acid.

To obtain substantially pure phytanic acid, i.e., with a purity by weight of more than 90%, preferably between 95-99%, the method of the present invention contemplates, once the previous phase separation has ended, performing a non-enzymatic hydrolysis step on the phytanate-CoA present in the solid phase resulting from the enzymatic thioesterification step, such that the phytanic acid can be separated from the remaining reaction components and be recovered in a liquid phase. Finally, and to obtain purified phytanic acid, the method of the present invention furthermore optionally contemplates performing a purification step on the phytanic acid present in said liquid fraction which allows eliminating the benzoic acid content present in the mixture and allows recovering purified phytanic acid.

On the other hand and for obtaining fats and oils free of phytanic acid, the present invention optionally contemplates performing a re-esterification step for re-esterifying the free fatty acids obtained in the liquid phase resulting from the separation step after the enzymatic thioesterification step, in order to reform or reconstruct triglycerides initially present in the food, such as milk or dairy products.

This phase will not be necessary if the initial food products are not going to be reconstituted or when the fatty acids free of phytanic acid can be incorporated directly in products for human or animal consumption, as is the case of fish oils with an omega-3 fatty acid content, such as EPA or DHA content, and preferably those the omega-3 fatty acid concentration of which is less than 65% by weight with respect to the total fatty acids in the sample, which can be used directly or together with excipients or antioxidants such as vitamin C or vitamin E, in products such as dietary, nutritional or cosmetic supplements.

Furthermore, this step would not be necessary either if the fatty acids are obtained from microbial biomass or microalgae, in which case use for human or animal consumption is not contemplated.

The different steps of the method are described below in detail:

1- Starting material hydrolysis step until obtaining free fatty acids

As treatment prior to phytanic acid-specific enzymatic thioesterification, the oils and fats are subjected to a hydrolysis step to convert them into free fatty acids.

This hydrolysis step can be performed through lipase action:

Lipolysis is a natural phenomenon of different endogenously produced lipases in mammals (phosphatidyl_lipase or LPL), or microbial leukocyte lipases, (e.g. *Pseudomonas fluorescens*) and other lipogenic enzymes involved in fatty acid synthesis and esterification process in the first few hours of milk storage. After milking, all these enzymes naturally increase free fatty acids (FFAs) in milk. Hydrolysis is commonly performed in the food industry with lipases (triacylglycerol acyl hydrolases, EC 3.1.1.3). Microbial lipases, particularly those of a fungal origin, particularly *Zygomycetes*, *Hyphomycetes*, *Ascomycetes*, *Coelomycetes* and yeasts, are also found abundantly in nature and are commercially significant. The following are mentioned by way of example: immobilized lipases of *Mucor spp.*, *Lipomyces starkeyi*, *Rhizopus spp.*, *Geotrichum candidum, Penicillium spp., Acremonium strictum, Candida rugosa, Humicola lanuginosa*, *Cunninghamella verticillata* and *Aspergillus spp.* The use of bacterial lipases, such as *Bacillus thermocatenulatus* or *Thermomyces lanuginosus* (the latter for producing biodiesel) lipases, *Candida rugosa* or *Mucor miehei* lipase (in the formation of monoglycerides in the food industry) is also viable. These lipases are often used in the milk industry, in manufacturing detergents, in bread baking, in the beverage industry, in biodiesel, in polymerization and in other chemical products of interest. The use of lipases is common in the milk industry and in the plant and animal oil industry, so the use thereof for performing the hydrolysis step in the method of the present invention would be viable.

Another viable option for the purpose of the present invention is to perform hydrolysis using conventional methods known in the state of the art, such as saponification by means of adding a base, such as potassium hydroxide, which alkalinizes at a pH between 8-9.5, to perform complete hydrolysis of the oil or fat. Said saponification step can optionally be followed by acidification.

In one embodiment of the present invention, a potassium hydroxide solution is added to the oil or fat to be treated to cause the saponification reaction, converting the fat or oil into reactive free phytanic acid and in a medium with an optimal pH for saponification and enzymatic activity (pH 8.7), heating the medium to a temperature between 40-90°C and adding alcohol (e.g.: ethanol) to start the reaction that will cause the complete hydrolysis and the formation of phytanic acid and fatty acid salts. Acidolysis is performed with acetic acid to obtain free fatty acids by separating potassium acetate. After complete hydrolysis, in some cases it may be necessary to separate glycerine or alcohol according to the starting material or oil; it may further be necessary to adapt the pH with a buffer until obtaining the pH that is optimal for subsequently producing the enzymatic thioesterification of this invention. The free fatty acids formed from the potassium-containing gelatinous broth of the aqueous phase are cooled or kept at the optimal reaction temperature of the following step, between 25-40°C, preferably 37°C.

2. Phytanic acid-specific enzymatic thioesterification step

This step occurs through the activity of an enzyme, phytanate-CoA ligase (PCL), which specifically activates phytanic acid to give rise to phytanate-CoA. The ligase preferably used in the method of the invention is the phytanate-CoA ligase, other names include AMP-forming phytanate-CoA ligase, mammal-specific phytanoyl CoA ligase (EC 6.2.1.24) from the liver of *Rattus norvegicus*, because its activity is optimal at the same temperature (25-40°C) and pH (8.7) as the saponification (hydrolysis) reaction of the preceding step. Said enzyme was obtained using the separation and purification method used by Muralidharan-FN & Muralidharan-VB. The enzyme PCL is found in the mitochondrial and microsomal fractions of various tissues (liver tissue, adipose tissue, heart tissue and kidney tissue) in mammals, including in humans, the function of which is to activate phytanic acid and synthesis phytanate-CoA in order to start the oxidation process ($\alpha$-oxidation or $\omega$-oxidation).

The enzyme PCL requires GTP, CTP or ATP for maximum activity, at a ratio of about (1-1.2 Mol) 3:1 g ATP/g phytanic acid, $Mg^{2+}$ (inhibition at high concentrations) and CoA at a ratio of about (1-1.2 Mol) 4:1 g CoA/g phytanic acid. The thiamine pyrophosphate is dose-dependent (e.g.: 4 $\mu$ml/L) to activate the ligase reaction. The reaction is equimolar, $Mg^{2+}$ (60 mM) or $Mn^{2+}$ (5 mM) being necessary. At an optimal pH of 8.7, it shows enzymatic activity of (value of Km=0.0286 mM) being inhibited by very high reaction product concentrations: AMP (value $K_i$ =11 mM). This allows not having to remove the AMP from the enzyme, it being able to be used in several cycles while the enzyme maintains activity and when the sample has a phytanic acid content < 1 mg/g. Complete thioesterification takes place equally in the two isomers found naturally in phytanic acid.

The reaction follows the following scheme:

$$ATP + phytanate + CoA \leftrightarrow AMP + diphosphate + phytanate\text{-} CoA$$

Nevertheless, this enzyme is not exclusive of other specific enzyme ligases that form phytanic acid thioesterification but do not esterify straight-chained fatty acids. Examples of other bacterial enzymes that can be used for the purpose of the present invention are: benzoate-CoA ligase (EC 6.2.1.25, also called benzoate-coenzyme A ligase, AMP-forming benzoyl-coenzyme A from *Thauera aromatica* strains K172 or DSM6984, or from *Azoarcus evansii* strain BH72 (optimal pH 7.8 at a temperature of 28°C), from *Clarkia breweri* with an optimal pH of 6.4, as well as α-proteobacteria, such as *Rhodopseudomonas palustris*, *Anaerobium bacterium*, or *Magnetospirillum sp*, having a comparable method and reaction medium requirements but an optimal pH of 7 and a lower temperature (22-44°C).

The reaction step is carried out by adding the enzyme PCL together with CoA and cofactors to favor the reaction. In a possible embodiment of the invention, the reaction medium where the reaction takes place is a saponified oil or fat in an alkaline medium at a pH between 7.8 and 8.7 (in the case of the enzyme PCL, the pH is 8.7), and at a temperature between 25°C and 37°C (in the case of the enzyme PCL, the temperature is between 35-37°C) under reduced pressure conditions < 10 Torr. In summary, the specific reaction conditions will always depend on the enzyme to be used.

CoA SH, thiamine pyrophosphate (4 mmol/L), ATP (1.2-1.5 per mol of phytanic acid) and a magnesium salt (i.e.: magnesium chloride, 60 mM) or a manganese salt (i.e.: manganese chloride, 5 mM) are also added.

A reaction tank with a stirrer was successfully used for the continuous formation of phytanic acid thioesters (phytanate-CoA) in less than 24 hours from the oil pool. Once the reaction medium is prepared, the enzyme is added, said enzyme being able to be in the form of a suspension (for example, Tris-HCl buffer ph 8.7), dry powder or immobilized on any support: nylon, amberlite, kaolinite, ceramic, etc., or being immobilized without being dried. The immobilization method is the most suitable. For this method, diatomaceous earth must be avoided as much as possible given its high phytanic acid content. A specific immobilization method is not necessary for the enzymatic treatment of the oil or fat, which usually has a higher reactivity than the free raw enzyme.

Depending on the type of enzyme, phytanic acid concentration, reaction medium, temperature, amount of water, the reaction time can range between 5 hours and 72 hours. Optimal conditions can be empirically estimated from the reaction parameters. The course of the reaction is monitored by analyzing the presence of phytanic acid. The phytanic acid determination is performed by gas chromatography, following the method for determining fatty acid composition described in the European Pharmacopoeia for oils rich in omega-3 suitable for determining phytanic acid and using a mass detector. It is essential in this invention for the degree of conversion to phytanate-CoA to be high and for the final phytanic acid concentration in the oil or fat to be less than 5 μg/g.

After the addition of the enzyme, the reaction mixture is stirred in a reaction tank with a stirrer or with an impeller. The stirring speed must be enough to maintain the suspension (200-500 rpm). According to the enzyme used, temperature is maintained between 20 and 50°C, until phytanic acid levels in the oil are less than 50 μg/g, preferably <5 μg/g and the conversion of phytanic acid to phytanate-CoA is virtually complete.

Two fractions are obtained as a result of the enzymatic thioesterification reaction: a solid fraction containing phytanate-CoA, ATP, AMP, $Mn_2Cl$, thiamine, enzyme and pyrophosphate, and a liquid fraction containing fatty acids substantially free of phytanic acid.

3.- Separation step for separating phytanate-CoA

The solid fraction containing phytanate-CoA and the liquid phase containing free fatty acids with a phytanic acid content less than 50 μg/g, preferably <5 μg/g, are separated in this step.

Once the enzymatic reaction in the preceding step is stopped by means of the process of removing the enzyme when desired phytanic acid levels have been reached, performing the determination by GC-MS chromatography, the solid and liquid phases obtained in the preceding step are separated by simple filtration or filtration/centrifugation cycles.

Given the physicochemical characteristics of all the reaction products and substrates, performing filtration and centrifugation cycles (5000-25000 rpm at 4°C) for carrying out this separation step is contemplated as a preferred option according to the present invention.

As described above, the solid fraction contains phytanate-CoA, AMP, pyrophosphate, thiamine pyrophosphate, $Cl_2Mg$, CoA, ATP, which remain in solid state at the reaction temperature thus producing a precipitate. As a result of this separation, on one hand the solid phase containing highly pure phytanate-CoA which could be used as a purified source of phytanic acid and CoA is obtained. On the other hand, and as a result of the separation step, a liquid phase made up of the free fatty acids with a phytanic acid content less than 50 $\mu$g/g, preferably <5 $\mu$g/g, is obtained.

This liquid phase can have a direct application in the food, pharmaceutical and cosmetics industries. In such case, an acid (for example, acetic acid) can be added to it to attain phase neutralization at pH 7 and to immediately proceed to separate the salts and water.

These free fatty acids can optionally be protected by adding a conventional, pharmaceutically acceptable or food grade antioxidant, such as ascorbic palmitate or tocopherols to conclude formulation in pharmaceutical, cosmetic or food products such as nutritional or dietary supplements.

When the fatty acids free of phytanic acid are to be used for human and/or animal consumption, this mixture of free fatty acids can also be subjected to an enzymatic esterification process for obtaining specific glycerides, as described below.

4.- Non-enzymatic hydrolysis step

Although the phytanate-CoA obtained in the solid phase as a result of the preceding separation is a product with a direct application in the chemical industry as a standard, a marker, etc., it is advisable to conclude the process of recovering phytanic acid in the form of a free acid for its most significant application as an additive in the chemical industry.

The solid fraction recovered after the preceding separation step, containing a mixture of phytanate-CoA, enzyme PCL, coenzyme, ATP, AMP, Mn$_2$Cl, thiamine and pyrophosphate, is subjected to non-enzymatic hydrolysis in a strongly alkaline medium, preferably pH 12, to obtain a new solid phase (CoA, PhAL and cofactors) and a liquid phase containing phytanic acid with a purity by weight of more than 90%.

Furthermore, phytanate-CoA does not present long-term stability, it being necessary to separate it from the enzyme in order to reuse it. Phytanic acid is liquid at room temperature, whereas phytanate-CoA is solid, so hydrolysis allows obtaining phytanic acid, CoA and enzyme in addition to cofactors, all separately. Both enzyme and cofactors, etc., can be reused in successive processes on the condition that enzyme activity is high. The activity half life of the ligase at room temperature is about 350 hours.

Complete hydrolysis occurs after 2 hours in a strongly alkaline medium, at a pH between 10 and 13, preferably pH 12, in a reference standard buffer solution, such as for example, CertiPUR® containing potassium phosphate and sodium dihydrogen/hydroxide. Phytanate-CoA is completely hydrolyzed into phytanic acid and CoA after 1-2 hours of incubation at 40°C. Phytanic acid is liquid at room temperature, being separated from the solid phase containing CoA by means of filtration/centrifugation.

5.- Purification step for purifying free Phytanic acid

The purpose of this step is to purify free phytanic acid from the liquid fraction containing phytanic acid obtained in the non-enzymatic hydrolysis step. This method does not act on the food or the source of phytanic acid, but rather on the liquid fraction containing phytanic acid obtained after the non-enzymatic hydrolysis step, made up of phytanic acid and benzoic acid at variable purities according to the concentration of benzoic acid in the raw material. This last step is optional because benzoic acid is an acid with a trace presence or with very low concentrations, obtained in the preceding step, usually with phytanic acid purities by weight of more than 90%, where the rest of the composition is mostly benzoic acid.

As previously discussed, it is also possible to use enzyme E.6.2.1.25, which is specific for benzoic acid or sodium benzoate, which are normally found at highly variable concentrations in foods naturally and added as preservatives and/or bactericides, in the enzymatic thioesterification step. By way of example, according to the WHO-Concise International Chemical Assessment Document 26 (Wibbertmann *et al*., 2005) first published by Sieber *et al.,* 1989, it is determined that benzoic acid is found in milk (traces-6 mg/kg), yogurt (12-40 mg/kg), cheese (traces-40 mg/kg), fish and marine products (30-2500 mg/kg), generally with a value of 100 mg/kg before preserving same.

In the process of obtaining refined fish oils, benzoic acid is usually separated from the non-saponifiable lipid and fatty acid fraction, so it is not common to find traces of benzoic acid therein. However, due to the differences in the refining processes and to the presence thereof in non-refined omega-3 fish oils, some milks, dairy fats, ruminant fats, it may be necessary to perform this step to separate benzoic acid from the liquid fraction obtained after non-enzymatic hydrolysis. A variable benzoic acid concentration in vegetable oils, microorganisms, algae, or marine animal (mammal) fats, is also expected to be found. In all these cases, it may be necessary to separate benzoic acid from phytanic acid to obtain a purified oil containing phytanic acid with a purity by weight of more than 90%.

As a result of this purification step, two highly pure solutions are obtained, one being a solution in benzoic acid used in the chemical and food industries as a preservative and bactericide, and another solution being a fraction with high phytanic acid purity.

There are conventional industrial methods known in the state of the art for performing this purification step for purifying phytanic acid, such as:

- HPLC
- Reactive extraction (D. Datta, S. Kumar, and K. L. Wasewar, "Reactive extraction of benzoic acid and pyridine-3-carboxylic acid using organophosphoric- and aminicextractant dissolved in binary diluent mixtures," 2011. J. Chem. Eng. Data, vol. 56, pp. 3367-3375; or revision of Kailas L. Wasewar. Reactive Extraction: An Intensifying Approach for Carboxylic Acid Separation. International Journal of Chemical Engineering and Applications, Vol. 3, No. 4, August 2012).
- Flash-Vacuum Distillation-Melt Crystallization (XU-Jiao,ZHANG-Weijiang,YANG-Tao, JIAO-Shujun,HU-Xuedong. Separation of Benzoic Acid Residue by Flash-Vacuum Distillation-Melt Crystallization. Trans. Tianjin Univ. 2009, 15: 288-293)

### 6. Optional fatty acid esterification step

This last step consists of re-esterifying the free fatty acids obtained in the liquid phase after the separation step after the specific thioesterification step into mono-, di-or triglycerides. The objective of this step is to reconstitute food products based on fats and oils for human and animal consumption.

Performing this step is particularly contemplated for food formation: dairy fats (milk cream, butter, milk, etc.) or fish oils or any food the fatty acids of which are originally presented in the form of a triglyceride.

Any method known by the person skilled in the art can be used to esterify the free fatty acids. A highly effective method is described in US patent application number US 20080114181 or in international patent application WO/2010/118761 belonging to the same applicant as the present invention.

A practical embodiment of the re-esterification step can be carried out using a Soxhlet condenser, where 5% styrene resin and the acid oil containing free fatty acids (200 g) and glycerol (50 g) are mixed, placing the mixture on a mechanical thermoshaker at 185 rpm and 40°C for 20 minutes. 80 ml of ethanol are added to the mixture and the temperature is increased to 60°C and shaking to 235 rpm. The reaction is maintained for 24 hours at atmospheric pressure. The method is performed in an inert atmosphere (nitrogen).

The re-esterification reaction can also be performed by means of any nonspecific lipase described in (WO/2010/118761), such as Novozyme 435 or the same one used in the first enzymatic hydrolysis or lipolysis step, so that it does not alter the fatty acid composition.

The following drawings and examples are provided below to illustrate the present invention. In no case do they intend to be limiting of the present invention.

### Examples

### Example 1: Method for separating phytanic acid from krill oil

### 1.1 Hydrolysis step by means of saponification followed by acidification for the formation of free fatty acids

1000 g of commercially available NKO® krill (*Euphausia superba*) oil (Neptune Technologies & Bioresources) with a phytanic acid content of 33210 $\mu$g/g, together with 335 g of KOH, 375 ml of water and 10 ml ethanol were added in a 5-liter reactor equipped with a stirrer to initiate the reaction at a temperature of 90°C in an inert atmosphere (nitrogen), stirring the mixture at 300 rpm for 1 hour. Three liters of 70% acetic acid (non-oxidizing acid) are added to the potassium broth obtained from fish oil and are mixed vigorously in an inert atmosphere (nitrogen). The potassium acetate is removed and the acidified fish oil is washed 5 times.

### 1.2 Phytanic acid-specific enzymatic thioesterification reaction step

Once all the fatty acids of the oil sample to be treated are free, the phytanic acid can react with the enzyme. In this case the enzyme phytanate-CoA ligase (EC 6.2.1.24) (10 g), which was obtained according to the method described by Muralidharan & Muralidharan, is selected for such purpose. The temperature of the oleaginous krill medium with the free fatty acids was reduced in the reactor and under vacuum conditions (<10 Torr) at 37°C, pH being maintained at 8.7. $Mn_2Cl$ hexahydrate (2.5 g), CoA (130 g), ATP (90 g), thiamine pyrophosphate (4 mmol) and phytanate-CoA ligase (EC 6.2.1.24) (10 g) were added to the reactor. The mixture was maintained under stirring at 300 rpm, at 37°C for 24 hours until obtaining phytanic acid levels of 0-5 $\mu$g/ml. By using the method for determining fatty acids by gas chromatography described in the European Pharmacopoeia for oils rich in omega-3 but adapted to phytanic acid and using a GC-MS detector, phytanic acid levels were obtained after 1 hour (6970 $\mu$g/g); 5 hours (712 $\mu$g/g); 12 hours (PhA: 61 $\mu$g/g), 24 hours (< 5 $\mu$g/g).

<u>1.3 Separation step for separating the solid phase containing phytanate-CoA</u>

Once the enzymatic thioesterification step has ended by means of removing the enzyme, the solid phase (260 g) and liquid phase are separated through a filtration/centrifugation cycle at 14000 rpm at 4°C for 10 minutes.

The liquid phase containing the free fatty acids with a phytanic acid content between 0-5 $\mu$g/g was neutralized with acetic acid until reaching pH 7, cooling the oil free of phytanic acid, removing water and ethanol using the rotary evaporator. An oil substantially free of phytanic acid is obtained without modifying the fatty acid composition.

<u>1.4 Non-enzymatic phytanate-CoA step. Phytanic acid purification</u>

To obtain highly pure phytanic acid and recover the enzyme and the products from the reaction, the solid phase separated in the preceding step, after dissolving the solid phase in 500 ml of ethanol and adding sodium hydroxide until reaching pH 12.5 at a temperature of 40°C, is subjected to alkaline hydrolysis, the complete hydrolysis of phytanate-CoA into phytanic acid and CoA occurring after two hours. Winterization at - 5°C for 12 hours was performed. Phytanic acid remains liquid at said temperature, the precipitate made up of CoA, phytanate-CoA-ligase, thiamine, AMP, pyrophosphate, ATP and $Mn_2Cl$ being separated by centrifugation and filtration (5000-25000 rpm at 4°C). Alcohol was removed from the liquid phase with a rotary evaporator until obtaining 40 ml of an oil in an alcohol solution of phytanic acid with a high purity (99.1% w/w) by the method for determining fatty acids by gas chromatography described in the European Pharmacopoeia for oils rich in omega-3 suitable for phytanic and using a GC-MS detector.

<u>1.5 Free fatty acid re-esterification step</u>

This is a completely optional process because free fatty acids are marketed due to their high bioavailability in the supplement industry. However, in this example a triglyceride was formed using a Soxhlet condenser where 5% styrene resin and the acid oil containing free fatty acids (200 g) and glycerol (50 g) are mixed, placing the mixture on a mechanical thermoshaker at 185 rpm and 40°C for 20 minutes. 80 ml of ethanol are added to the mixture and the temperature is increased to 60°C and shaking to 235 rpm. The reaction is maintained for 24 hours at atmospheric pressure. The method is performed in an inert atmosphere (nitrogen).

<u>Example 2: Method for separating phytanic acid from a dairy product: butter</u>

The same process used in Example 1 was performed but this time starting from 1000 g of commercially available butter, with a phytanic acid concentration of 4530 $\mu$g/g. $Mn_2Cl$ hexahydrate (2.1 g), CoA (20 g), ATP (13 g), thiamine (4 mmol/L) and phytanate-CoA ligase (10 g) were added to the reactor for enzymatic treatment. Phytanic acid levels were obtained after 1 hour (276 $\mu$g/g); 5 hours (87 $\mu$g/g); 12 hours (PhA: 17 $\mu$g/g), 24 hours (< 5 $\mu$g/g). The oil without phytanic acid was neutralized and phytanate-CoA hydrolysis was performed, obtaining 10 ml of an alcohol solution of phytanic acid (99.3% w/w).

Finally, the re-esterification step of Example 1 was performed to reconstruct the butter food product.

<u>Example 3: Method for separating phytanic acid from non-refined tuna fish oil for obtaining fish oils with a concentration less than 65% by weight of omega-3 fatty acids and a phytanic acid content less than 50 $\mu$g/g, and less than 5 $\mu$g/g</u>

Non-refined fish oils can have highly variable phytanic acid levels (e.g.: phytanic acid from tuna fish oil: 10320 $\mu$g/g).

The same process used in Example 1 , steps 1.1-1.3, was used but this time starting with 1000 g of concentrated tuna fish oil with 50% omega-3 fatty acid triglyceride, commercially available, Incromega® 500TG (Croda) DHA, with a phytanic acid concentration of 1650 $\mu$g/g. $Mn_2Cl$ hexahydrate (2 g), CoA (6.5 g), ATP (4.5 g), thiamine (4 mmol/L) and phytanate-CoA ligase (10 g) were added to the reactor for enzymatic treatment. As a result of this method, fish oils (tuna) with omega-3 fatty acid concentrations less than 65% by weight with respect to the total fatty acids of the sample were obtained with a phytanic acid content after 1 hour (340 $\mu$g/g); 5 hours (93 $\mu$g/g); 12 hours (PhA: 23 $\mu$g/g), 24 hours (< 5 $\mu$g/g). 5 ml of an alcohol solution of phytanic acid (98.3% w/w) were obtained by means of phytanate-CoA hydrolysis.

<u>Example 4: Method for separating phytanic acid from a microbial biomass: diatoms (*Biddulphia sinensis*)</u>

The same process used in Example 1 was performed but this time starting from 1000 g of *Biddulphia sinensis*, with a phytanic acid concentration of 4.6 mg/g. $Mn_2Cl$ hexahydrate (2 g), CoA (0.6 g), ATP (0.4 g), thiamine (4 mmol/L) and phytanate-CoA ligase (10 g) were added to the reactor for enzymatic treatment. Phytanic acid levels were obtained after 1 hour (1832 $\mu$g/g); 5 hours (< 5 $\mu$g/g); 12 hours (PhA: <5 $\mu$g/g), 24 hours (< 5 $\mu$g/g). 34 ml of an alcohol solution of PhA with (98.9% w/w) was obtained by means of phytanate-CoA hydrolysis.

As a conclusion of the practical examples that were carried out, a table is enclosed below which shows the phytanic acid content obtained in the different samples subjected to the method of the present invention and recovered in step c).

Table comparing PhA concentrations (μg/g):

|  | t0 | t1 (1h) | t2 (5h) | t3 (12h) | t4 (24h) |
|---|---|---|---|---|---|
| Krill oil (Example 1) | 33210 | 6970 | 712 | 61 | < 5 |
| Butter (Example 2) | 4530 | 276 | 87 | 17 | < 5 |
| Non-refined tuna fish oil (Example 3) | 1650 | 340 | 93 | 23 | < 5 |
| Microalgae oil (Example 4) | 134 | 26 | < 5 | < 5 | < 5 |

**References**

Allen, N. E., Grace, P. B., Ginn, A., Travis, R. C., Roddam, A. W., Appleby, P. N., & Key, T. (2008). Phytanic acid: measurement of plasma concentrations by gas-liquid chromatography-mass spectrometry analysis and associations with diet and other plasma fatty acids. British Journal of Nutrition, 99(3), 653.

Astarita, G., Jung, K. M., Berchtold, N. C., Nguyen, V. Q., Gillen, D. L., Head, E., ... & Piomelli, D. (2010). Deficient liver biosynthesis of docosahexaenoic acid correlates with cognitive impairment in Alzheimer's disease. PLoS One, 5(9), e12538.

Atshaves BP, Storey SM, Petrescu A, Greenberg CC, Lyuksyutova OI, Smith III R, Schroeder F. Expression of fatty acid binding proteins inhibits lipid accumulation and alters toxicity in L cell fibroblasts. Am J Physiol Cell Physiol 2002; 283:C688-C703.

Atshaves, B. P., Schroeder, F., Starodub, O., Boedeker, A. L., Smith Iii, R. R., Roths, J. B., ... & Kier, A. B. (2001). Expression of liver fatty acid binding protein alters growth and differentiation of embryonic stem cells. Molecular and cellular biochemistry, 219(1-2), 127-138.

Britton-TC, Gibberd-FB, Clemens-ME, Billimoria-JD, Sidey-MC. The significance of plasma phytanic acid levels in adults. J Neurol Neurosurg Psychiatry 1989;52(7):891-894.

Brown, P., Mei, G., Gibberd, F. B., Burston, D., Mayne, P. D., McClinchy, J. E., & Sidey, M. (1993). Diet and Refsum's disease. The determination of phytanic acid and phytol in certain foods and the application of this knowledge to the choice of suitable convenience foods for patients with Refsum's disease. Journal of Human Nutrition and Dietetics, 6(4), 295-305.

Ferdinandusse, S., Denis, S., Clayton, P. T., Graham, A., Rees, J. E., Allen, J. T., ... & Wanders, R. J. (2000). Mutations in the gene encoding peroxisomal α-methylacyl-CoA racemase cause adult-onset sensory motor neuropathy. Nature genetics, 24(2), 188-191.

Fingerhut R, Schmitz W, Garavaglia B, Reichmann H, Conzelmann E. Impaired degradation of phytanic acid in cells from patients with mitochondriopathies: evidence for the involvement of ETF and the respiratory chain in phytanic acid alpha-oxidation. J Inherit Metab Dis 1994; 17(5):527-532.

Hashimoto, T., Shimizu, N., Kimura, T., Takahashi, Y., & Ide, T. (2006). Polyunsaturated fats attenuate the dietary phytol-induced increase in hepatic fatty acid oxidation in mice. The Journal of nutrition, 136(4), 882-886.

Higashihara, E., Itomura, M., Terachi, T., Matsuda, T., Kawakita, M., Kameyama, S., ... & Takada, H. (2010). Effects of eicosapentaenoic acid on biochemical failure after radical prostatectomy for prostate cancer. In Vivo, 24(4), 561-565.

Kahlert, S., Schönfeld, P., & Reiser, G. (2005). The Refsum disease marker phytanic acid, a branched chain fatty acid, affects $Ca^{2+}$ homeostasis and mitochondria, and reduces cell viability in rat hippocampal astrocytes. Neurobiology of disease, 18(1), 110-118.

Lazarow PB, Moser HW. Disorders of peroxisome biogenesis. In: Seriver CR, et al (eds.). This metabolic and molecular bases of inherited disease, vol II. McGraw-Hill. New York, pp 2287-2324.

Lizard, G., Rouaud, O., Demarquoy, J., Cherkaoui-Malki, M., & Iuliano, L. (2012). Potential Roles of Peroxisomes in Alzheimer's Disease and in Dementia of the Alzheimer's Type. Journal of Alzheimer's disease, 29(2), 241-254.

Lloyd MD, Darley DJ, Wierzbicki AS, Threadgill MD. (2008). Alpha-methylacyl-CoA racemase-an 'obscure' metabolic enzyme takes centre stage. FEBS J 275: 1089-110.

Mobley JA, Leav I, Zielie P, Wotkovitz C, Evans J, Lam YV, L'Esperance BS, Jiang Z, Ho SM. Branched fatty acids in dairy and beef products markedly enhance $\alpha$-methylacyl-CoA Racemase expression in prostate cancer cells in vitro. Cancer Epidemiology, Biomarkers & Prevention 2003; 12:775-783.

Mönning, G., Wiekowski, J., Kirchhof, P., STYPMANN, J., Plenz, G., Fabritz, L. & Seedorf, U. (2004). Phytanic Acid Accumulation Is Associated with Conduction Delay and Sudden Cardiac Death in Sterol Carrier Protein-2/Sterol Carrier Protein-x Deficient Mice. Journal of cardiovascular electrophysiology, 15(11), 1310-1316.

Muralidharan, F. N., & Muralidharan, V. B. (1986). Phytanoyl-CoA ligase activity in rat liver. Biochemistry international, 13(1), 123.

Ollberding, N. J., Aschebrook-Kilfoy, B., Caces, D. B. D., Wright, M. E., Weisenburger, D. D., Smith, S. M., & Chiu, B. C. H. (2013). Phytanic acid and the risk of non-Hodgkin lymphoma. Carcinogenesis, 34(1), 170-175.

Pollit-RJ. Disorders of Mitochondrial long-chain fatty acid oxidation. J Inherit Metab Dis. 1995; 18(4):473-90.

Powers JM, Kenjarski TP, Moser AB, Moser HW. Cerebellar atrophy in chronic rhizomelic chondrodysplasia punctata: a potential role for phytanic acid and calcium in the death of its Purkinje cells. Acta Neuropathol (Berl) 1999; 98(2):129-134.

Schaefer EJ, Robins SJ, Patton GM, et al. Red blood cell membrane phosphatidylethanolamine fatty acid content in various forms of retinitis pigmentosa. J Lipid Res 1995;36:1427-33.

Steinberg D. Phytanic acid storage disease (Refsum's disease). In Metabolic Basis of Inherited Disease. Edited by Stanbury JB, Wyngarden JB, Fredericksen DS, Goldstein JL, Brown MS. New York: McGraw Hill; 5. 1983:731-747.

Takemoto-Y, Suzuki-Y, Horibe-R, Shimozawa-N, Wanders-RJ, Kondo-N. Gas chromatography/mass spectrometry analysis of very long chain fatty acids, docosahexaenoic acid, phytanic acid and plasmalogen for screening of perxisomal disorders. Brain Dev. 2003; 25(7):481-7.

Thornburg, T., Turner, A. R., Chen, Y. Q., Vitolins, M., Chang, B., & Xu, J. (2006). Phytanic acid, AMACR and prostate cancer risk.

Wallström, P., Bjartell, A., Gullberg, B., Olsson, H., & Wirfält, E. (2007). A prospective study on dietary fat and incidence of prostate cancer (Malmö, Sweden). Cancer Causes & Control, 18(10), 1107-1121.

Wanders RJA, Waterham HR, Leroy BP. Refsum disease. In: Pagon RA, Bird TC, Dolan CR, Stephens K editors. GeneReviews [Internet], Sealttle (WA): University of Washington, Sealttle; 1993-2006 [updated 2010].

Warneer K, Eskin M. Methods of Assess Quality and Stability of Oil and Fat-Containing Foods". Kathleen Warner and N.A. Michael Eskin Editors. 1995.

Watkins PA, Moser AB, Toomer CB, steinberg SJ, Moser HW, Karaman MW, Ramaswamy K, Siegmund KD, Lee DR, Ely JJ, Ryder OA, Hacia JG. Identification of differences in human and great ape phytanic acid metabolism that could influence gene expression profiles and physiological functions. BMC Physiology 2010; 10(19):1-10.

Wierzbicki AS. Biochemical disorders affecting phytanic acid alpha-oxidation: a review. Biochem Soc Trans 2007; 35 (Pt 5): 881-886.

Wierzbicki AS, Lloyd MD, Schofield CJ, Feher MD, Gibberd FB. Refsum's disease: a peroxisomal disorder affecting phytanic acid alpha-oxidation. J Neurochem 2002; 80 (5): 727-735.

Wierzbicki, A. S. (2007). Peroxisomal disorders affecting phytanic acid alpha-oxidation: a review. Biochemical Society Transactions, 35(Pt 5), 881.

Wright, M. E., Bowen, P., Virtamo, J., Albanes, D., & Gann, P. H. (2012). Estimated phytanic acid intake and prostate cancer risk: a prospective cohort study. International Journal of Cancer, 131(6), 1396-1406.

Xu J, Thornburg T, Turner AR, Vitolins M, Case D, Shadle J, Hinson L, Sun J, Liu W, Chang B, Adams TS, Zheng SL, Torti FM. Serum levels of phytanic acid are associated with prostate cancer. Prostate 2005; 63:209-214.

Xu, J., Thornburg, T., Turner, A. R., Vitolins, M., Case, D., Shadle, J., ... & Torti, F. M. (2005). Serum levels of phytanic acid are associated with prostate cancer risk. The Prostate, 63(3), 209-214.

## Claims

1. A method for separating phytanic acid from oils and fats containing phytanic acid, **characterized by** comprising a phytanic acid-specific enzymatic thioesterification step which results in obtaining a solid phase comprising phytanate-CoA and a liquid fraction comprising fatty acids free of phytanic acid.

2. The method according to claim 1, **characterized in that** the enzymatic thioesterification step takes place in the presence of the enzyme phytanate-CoA ligase (EC 6.2.1.24).

3. The method according to claim 1, **characterized in that** the enzymatic thioesterification step takes place in the presence of the enzyme benzoate-CoA ligase (EC 6.2.1.25).

4. The method according to claims 1 to 3, **characterized in that** the enzymatic thioesterification step is carried out at a temperature between 25-37°C and at a pH of 7.8 to 8.7.

5. The method according to the preceding claims, **characterized in that** it comprises a starting oil and fat hydrolysis step to obtain free fatty acids prior to the thioesterification step.

6. The method according to the preceding claims, **characterized in that** the hydrolysis step is performed through saponification or by means of a lipase.

7. The method according to claim 6, **characterized in that** the hydrolysis step consists of saponification in the presence of potassium hydroxide followed by acidification in the presence of acetic acid.

8. The method according to the preceding claims, **characterized in that** it comprises a separation step after the thioesterification step for separating the solid fraction containing phytanate-CoA and the liquid fraction containing fatty acids with a phytanic acid content less than 50 $\mu$g/g, obtained in the thioesterification step.

9. The method according to claim 8, **characterized in that** the separation step is performed through centrifugation and filtration cycles.

10. The method according to claims 8 and 9 for obtaining phytanic acid with a purity by weight of more than 90%, **characterized in that** the solid fraction of phytanate-CoA is subjected to non-enzymatic hydrolysis in alkaline medium for obtaining a liquid phase containing phytanic acid with a purity by weight of 90%.

11. The method according to claim 10, **characterized by** additionally comprising a purification step for purifying free phytanic acid contained in the liquid phase to separate it from benzoic acid.

12. The method according to claims 8 and 9 for obtaining oils and fats with a phytanic acid content less than 50 $\mu$g/g, **characterized in that** the liquid fraction is subjected to a re-esterification step for re-esterifying free fatty acids into mono-, di- and/or triglycerides.

13. The method according to claim 12, **characterized in that** the re-esterification of free fatty acids gives rise to fish oils and/or milk and/or dairy products with a phytanic acid content less than 50 µg/g.

14. Use of fatty acids with a phytanic acid content less than 50 µg/g obtained through the method of the preceding claims 1 to 13 for human and animal consumption in the food, pharmaceutical and cosmetics industries.

15. Use of fish oils with an omega-3 fatty acid concentration less than 65% by weight with respect to the total fatty acids in the sample and a phytanic acid content less than 50 µg/g obtained through the method according to the preceding claims 1 to 9 and 12-13 for human and animal consumption in the food, pharmaceutical and cosmetics industries.

16. Use of milk and dairy products with a phytanic acid content less than 50 µg/g obtained through the method of the preceding claims 1 to 9 and 12-13 for human and animal consumption.

17. Use of phytanic acid with a purity by weight of more than 90% obtained through the method of the preceding claims 1 to 13 for use as an additive in the chemical, cosmetics and pharmaceutical industries.

Europäisches
Patentamt

European
Patent Office

Office européen
des brevets

# EUROPEAN SEARCH REPORT

Application Number

EP 13 38 2494

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2010/118761 A1 (EOLAS SCIENCE LTD [IE]; CELA LOPEZ JOSE MANUEL [IE]) 21 October 2010 (2010-10-21) * claims * | 4-17 | INV. C11B3/00 C12P7/40 C12P7/64 |
| X,D | US 2011/033595 A1 (KRUMBHOLZ RUDOLF [DE] ET AL) 10 February 2011 (2011-02-10) * claims * | 14-17 | |
| X | WO 2010/139003 A1 (COMMW SCIENT IND RES ORG [AU]; MOGHADDAM MINOO JALILI [AU]; DRUMMOND C) 9 December 2010 (2010-12-09) * page 64, lines 2- 16; claims * | 17 | |
| A | JANSEN G A ET AL:  "Alpha-Oxidation", BIOCHIMICA ET BIOPHYSICA ACTA. MOLECULAR CELL RESEARCH, ELSEVIER SCIENCE PUBLISHERS, AMSTERDAM, NL, vol. 1763, no. 12, 1 December 2006 (2006-12-01), pages 1403-1412, XP027896775, ISSN: 0167-4889 [retrieved on 2006-12-01] * the whole document * | 1-13 | TECHNICAL FIELDS SEARCHED (IPC) C11B C12P |

The present search report has been drawn up for all claims

| Place of search | Date of completion of the search | Examiner |
|---|---|---|
| Berlin | 12 May 2014 | Boeker, Ruth |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.......................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 13 38 2494

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2014

| Patent document cited in search report | Publication date | Patent family member(s) | Publication date |
|---|---|---|---|
| WO 2010118761 A1 | 21-10-2010 | AU 2009344374 A1<br>CA 2756528 A1<br>CN 102438468 A<br>EP 2429317 A1<br>JP 2012523823 A<br>KR 20120039521 A<br>US 2012053242 A1<br>WO 2010118761 A1 | 27-10-2011<br>21-10-2010<br>02-05-2012<br>21-03-2012<br>11-10-2012<br>25-04-2012<br>01-03-2012<br>21-10-2010 |
| US 2011033595 A1 | 10-02-2011 | NONE | |
| WO 2010139003 A1 | 09-12-2010 | NONE | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0110809 A
- EP 1157692 A
- US 2011033595 A
- EP 2464240 A
- WO 2010118761 A
- US 20080114181 A

**Non-patent literature cited in the description**

- **D. DATTA ; S. KUMAR ; K. L. WASEWAR.** Reactive extraction of benzoic acid and pyridine-3-carboxylic acid using organophosphoric- and aminicextractant dissolved in binary diluent mixtures. *J. Chem. Eng. Data,* 2011, vol. 56, 3367-3375
- **KAILAS L. WASEWAR.** Reactive Extraction: An Intensifying Approach for Carboxylic Acid Separation. *International Journal of Chemical Engineering and Applications,* August 2012, vol. 3 (4
- **XU-JIAO,ZHANG-WEIJIANG ; YANG-TAO ; JIAO-SHUJUN,HU-XUEDONG.** *Separation of Benzoic Acid Residue by Flash-Vacuum Distillation-Melt Crystallization,* 2009, vol. 15, 288-293
- **ALLEN, N. E. ; GRACE, P. B. ; GINN, A. ; TRAVIS, R. C. ; RODDAM, A. W. ; APPLEBY, P. N. ; KEY, T.** Phytanic acid: measurement of plasma concentrations by gas-liquid chromatography-mass spectrometry analysis and associations with diet and other plasma fatty acids. *British Journal of Nutrition,* 2008, vol. 99 (3), 653
- **ASTARITA, G. ; JUNG, K. M. ; BERCHTOLD, N. C. ; NGUYEN, V. Q. ; GILLEN, D. L. ; HEAD, E. ; PIOMELLI, D.** Deficient liver biosynthesis of docosahexaenoic acid correlates with cognitive impairment in Alzheimer's disease. *PLoS One,* 2010, vol. 5 (9), e12538
- **ATSHAVES BP ; STOREY SM ; PETRESCU A ; GREENBERG CC ; LYUKSYUTOVA OI ; SMITH III R ; SCHROEDER F.** Expression of fatty acid binding proteins inhibits lipid accumulation and alters toxicity in L cell fibroblasts. *Am J Physiol Cell Physiol,* 2002, vol. 283, C688-C703
- **ATSHAVES, B. P. ; SCHROEDER, F. ; STARODUB, O. ; BOEDEKER, A. L. ; SMITH III, R. R. ; ROTHS, J. B. ; KIER, A. B.** Expression of liver fatty acid binding protein alters growth and differentiation of embryonic stem cells. *Molecular and cellular biochemistry,* vol. 219 (1-2), 127-138
- **BRITTON-TC ; GIBBERD-FB ; CLEMENS-ME ; BILLIMORIA-JD ; SIDEY-MC.** The significance of plasma phytanic acid levels in adults. *J Neurol Neurosurg Psychiatry,* 1989, vol. 52 (7), 891-894
- **BROWN, P. ; MEI, G. ; GIBBERD, F. B. ; BURSTON, D. ; MAYNE, P. D. ; MCCLINCHY, J. E. ; SIDEY, M.** Diet and Refsum's disease. The determination of phytanic acid and phytol in certain foods and the application of this knowledge to the choice of suitable convenience foods for patients with Refsum's disease. *Journal of Human Nutrition and Dietetics,* 1993, vol. 6 (4), 295-305
- **FERDINANDUSSE, S. ; DENIS, S. ; CLAYTON, P. T. ; GRAHAM, A. ; REES, J. E. ; ALLEN, J. T. ; WANDERS, R. J.** Mutations in the gene encoding peroxisomal $\alpha$-methylacyl-CoA racemase cause adult-onset sensory motor neuropathy. *Nature genetics,* 2000, vol. 24 (2), 188-191
- **FINGERHUT R ; SCHMITZ W ; GARAVAGLIA B ; REICHMANN H ; CONZELMANN E.** Impaired degradation of phytanic acid in cells from patients with mitochondriopathies: evidence for the involvement of ETF and the respiratory chain in phytanic acid alpha-oxidation. *J Inherit Metab Dis,* 1994, vol. 17 (5), 527-532
- **HASHIMOTO, T ; SHIMIZU, N. ; KIMURA, T. ; TAKAHASHI, Y. ; IDE, T.** Polyunsaturated fats attenuate the dietary phytol-induced increase in hepatic fatty acid oxidation in mice. *The Journal of nutrition,* 2006, vol. 136 (4), 882-886
- **HIGASHIHARA, E. ; ITOMURA, M. ; TERACHI, T. ; MATSUDA, T ; KAWAKITA, M. ; KAMEYAMA, S. ; TAKADA, H.** Effects of eicosapentaenoic acid on biochemical failure after radical prostatectomy for prostate cancer. *In Vivo,* 2010, vol. 24 (4), 561-565
- **KAHLERT, S. ; SCHÖNFELD, P. ; REISER, G.** The Refsum disease marker phytanic acid, a branched chain fatty acid, affects $Ca^{2+}$ homeostasis and mitochondria, and reduces cell viability in rat hippocampal astrocytes. *Neurobiology of disease,* 2005, vol. 18 (1), 110-118
- Disorders of peroxisome biogenesis. **LAZAROW PB ; MOSER HW. et al.** This metabolic and molecular bases of inherited disease. McGraw-Hill, vol. II, 2287-2324

- **LIZARD, G. ; ROUAUD, O. ; DEMARQUOY, J. ; CHERKAOUI-MALKI, M. ; IULIANO, L.** Potential Roles of Peroxisomes in Alzheimer's Disease and in Dementia of the Alzheimer's Type. *Journal of Alzheimer's disease,* 2012, vol. 29 (2), 241-254
- **LLOYD MD ; DARLEY DJ ; WIERZBICKI AS ; THREADGILL MD.** Alpha-methylacyl-CoA racemase-an 'obscure' metabolic enzyme takes centre stage. *FEBS J,* 2008, vol. 275, 1089-110
- **MOBLEY JA ; LEAV I ; ZIELIE P ; WOTKOVITZ C ; EVANS J ; LAM YV ; L'ESPERANCE BS ; JIANG Z ; HO SM.** Branched fatty acids in dairy and beef products markedly enhance α-methylacyl-CoA Racemase expression in prostate cancer cells in vitro. *Cancer Epidemiology, Biomarkers & Prevention,* vol. 12, 775-783
- **MÖNNING, G ; WIEKOWSKI, J. ; KIRCHHOF, P. ; STYPMANN, J. ; PLENZ, G. ; FABRITZ, L ; SEEDORF, U.** Phytanic Acid Accumulation Is Associated with Conduction Delay and Sudden Cardiac Death in Sterol Carrier Protein-2/Sterol Carrier Protein-x Deficient Mice. *Journal of cardiovascular electrophysiology,* 2004, vol. 15 (11), 1310-1316
- **MURALIDHARAN, F. N. ; MURALIDHARAN, V. B.** Phytanoyl-CoA ligase activity in rat liver. *Biochemistry international,* 1986, vol. 13 (1), 123
- **OLLBERDING, N. J. ; ASCHEBROOK-KILFOY, B ; CACES, D. B. D. ; WRIGHT, M. E. ; WEISENBURGER, D. D. ; SMITH, S. M. ; CHIU, B. C. H.** Phytanic acid and the risk of non-Hodgkin lymphoma. *Carcinogenesis,* 2013, vol. 34 (1), 170-175
- **POLLIT-RJ.** Disorders of Mitochondrial long-chain fatty acid oxidation. *J Inherit Metab Dis.,* 1995, vol. 18 (4), 473-90
- **POWERS JM ; KENJARSKI TP ; MOSER AB ; MOSER HW.** Cerebellar atrophy in chronic rhizomelic chondrodysplasia punctata: a potential role for phytanic acid and calcium in the death of its Purkinje cells. *Acta Neuropathol,* 1999, vol. 98 (2), 129-134
- **SCHAEFER EJ ; ROBINS SJ ; PATTON GM et al.** Red blood cell membrane phosphatidylethanolamine fatty acid content in various forms of retinitis pigmentosa. *J Lipid Res,* 1995, vol. 36, 1427-33
- Phytanic acid storage disease (Refsum's disease. **STEINBERG D.** Metabolic Basis of Inherited Disease. McGraw Hill, 1983, vol. 5, 731-747
- **TAKEMOTO-Y ; SUZUKI-Y ; HORIBE-R ; SHIMOZAWA-N ; WANDERS-RJ ; KONDO-N.** Gas chromatography/mass spectrometry analysis of very long chain fatty acids, docosahexaenoic acid, phytanic acid and plasmalogen for screening of perxisomal disorders. *Brain Dev.,* 2003, vol. 25 (7), 481-7
- **THORNBURG, T ; TURNER, A. R. ; CHEN, Y. Q. ; VITOLINS, M. ; CHANG, B. ; XU, J.** *Phytanic acid, AMACR and prostate cancer risk,* 2006
- **WALLSTRÖM, P. ; BJARTELL, A. ; GULLBERG, B. ; OLSSON, H. ; WIRFÄLT, E.** A prospective study on dietary fat and incidence of prostate cancer (Malmö, Sweden. *Cancer Causes & Control,* 2007, vol. 18 (10), 1107-1121
- Refsum disease. **WANDERS RJA ; WATERHAM HR ; LEROY BP.** GeneReviews
- **WARNEER K ; ESKIN M.** Methods of Assess Quality and Stability of Oil and Fat-Containing Foods. 1995
- **WATKINS PA ; MOSER AB ; TOOMER CB ; STEINBERG SJ ; MOSER HW ; KARAMAN MW ; RAMASWAMY K ; SIEGMUND KD ; LEE DR ; ELY JJ.** Identification of differences in human and great ape phytanic acid metabolism that could influence gene expression profiles and physiological functions. *BMC Physiology,* 2010, vol. 10 (19), 1-10
- **WIERZBICKI AS.** Biochemical disorders affecting phytanic acid alpha-oxidation: a review. *Biochem Soc Trans,* 2007, vol. 35, 881-886
- **WIERZBICKI AS ; LLOYD MD ; SCHOFIELD CJ ; FEHER MD ; GIBBERD FB.** Refsum's disease: a peroxisomal disorder affecting phytanic acid alpha-oxidation. *J Neurochem,* 2002, vol. 80 (5), 727-735
- **WIERZBICKI, A. S.** Peroxisomal disorders affecting phytanic acid alpha-oxidation: a review. *Biochemical Society Transactions,* 2007, vol. 35, 881
- **WRIGHT, M. E. ; BOWEN, P. ; VIRTAMO, J. ; ALBANES, D. ; GANN, P. H.** Estimated phytanic acid intake and prostate cancer risk: a prospective cohort study. *International Journal of Cancer,* 2012, vol. 131 (6), 1396-1406
- **XU J ; THORNBURG T ; TURNER AR ; VITOLINS M ; CASE D ; SHADLE J ; HINSON L ; SUN J ; LIU W ; CHANG B.** Serum levels of phytanic acid are associated with prostate cancer. *Prostate,* 2005, vol. 63, 209-214
- **XU, J. ; THORNBURG, T. ; TURNER, A. R. ; VITOLINS, M ; CASE, D. ; SHADLE, J. ; TORTI, F. M.** Serum levels of phytanic acid are associated with prostate cancer risk. *The Prostate,* 2005, vol. 63 (3), 209-214